# EUROPEAN PATENT APPLICATION

(11) **EP 0 581 165 A2**
(43) Date of publication of application: **02.02.1994**
(21) Application number: 93111579.4
(22) Date of filing: 20.07.1993
(51) Int. Cl.: C07D 453/02, A61K 31/435, C07D 451/04

(54) **Acrylamide derivatives as antirussive agent**

(30) Priority: 29.07.1992 IT MI921851
(71) Applicant: DOMPE' FARMACEUTICI S.p.A., I-20122 Milano (IT)
(72) Inventor: Mantovanini, Marco, I-20154 Milano (IT); Giani, Roberto, I-20085 Locate Triulzi Milano (IT); Bestetti, Alessandro, I-20089 Basiglio Milano (IT); Lavezzo, Antonio, I-20075 Lodi Milano (IT); Omini, Claudio, I-20060 Bussero Milano (IT)
(74) Representative: Beneduce, Gianna

(57) **Abstract**

Acrylic amides having the formula 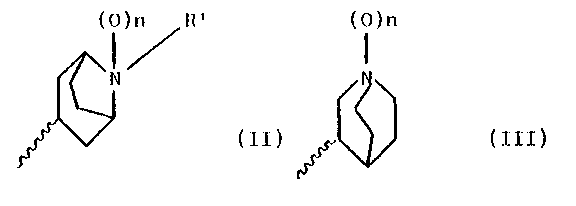
wherein Ar represents a pyridyl radical or an optionally substituted benzyl radical; Ra represents hydrogen, lower alkyl, phenyl, cyano; Q represents the tropyl or quinuclidinyl radicals optionally N-oxo substituted, are described.

The compounds of formula (I) show antitussive activity.

## Description

The object of the present invention relates to pharmacological active derivatives of the acrylic acid, the process for their preparation and the pharmaceutical compositions containing them.

More particularly the derivatives of the acrylic acid object of the invention, are represented by the acrylic amides having the structure formula 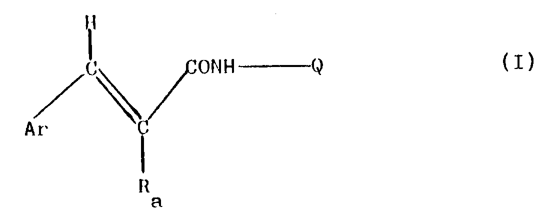
wherein
- Ar: represents a pyridyl radical or a benzene ring which optionally may be substituted with one or more halogen atoms or one or more groups selected among lower alkyl, lower haloalkyl, lower alkoxy, phenoxy, hydroxy, acyloxy, acylamino, amino and nitro,
- Rₐ: represents hydrogen, lower alkyl, phenyl, cyano;
- Q: represents a bicyclic octylamine radical selected among the tropyl and quinuclidinyl radicals having respectively the structures

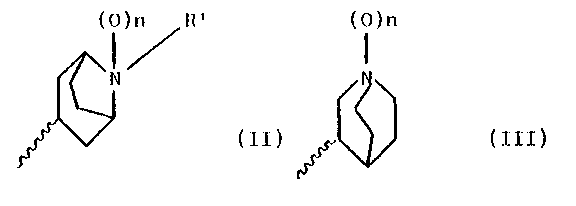

wherein
- R': represents a lower alkyl group and
- n: represents 0 or 1, provided that n=0 when Rₐ is cyano and/or Ar is pyridyl.

The pharmaceutically acceptable addition salts of the compounds (I) constitute a further object of the invention: hydrochlorides, hydrobromides, hydroiodides, alkyl and arylsulphonates, succinates, tartrates and citrates are particularly cited among them.

With the term lower alkyl and lower alkoxy, it is meant to indicate an alkyl radical or a alkoxy radical containing from 1 to 4 carbon atoms. Methyl radical and methoxy radical are preferred among these. As halogen atom, is meant fluorine, chlorine and bromine. The lower haloalkyl group is a haloalkyl group containing from 1 to 4 carbon atoms: trichloromethyl and trifluoromethyl are preferred among these.

The acyl group is generally constituted by an acyl radical containing from 1 to 7 carbon atoms: the acetyl radical is preferred among these. The pyridyl radical can be represented by α-pyridyl or β-pyridyl or γ-pyridyl radical.

Accordingly to the configuration of the tropyl and quinuclidinyl radical, the compounds (I) may assume the configuration endo, exo or mixture of the same.

The compounds of formula (I) are particularly preferred in which Ar represents the benzene ring and particularly the derivatives of the cinnamic amide 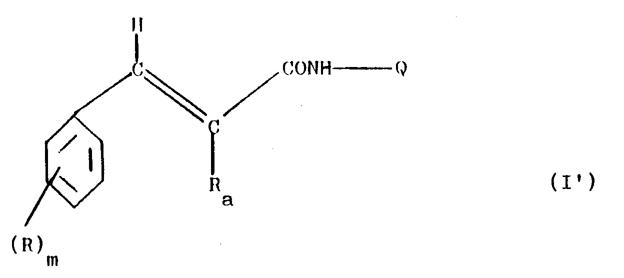
wherein R represents a hydrogen, florine, chlorine, bromine atom or a lower alkyl, lower alkoxy, hydroxy, phenoxy, amino, acylamino and nitro radical; m is an integer from 1 to 5 and Rₐ and Q have the previously described meanings.

The compounds (I) object of the invention are endowed with an interesting antitussive activity which has demonstrated to be without effects on the central nervous system.

The compounds of formula (I) in which n is equal to 0 are prepared starting from an acryl derivative (IV) and from octylamine (V), optionally in a suitable addition salt form, according to the reaction: 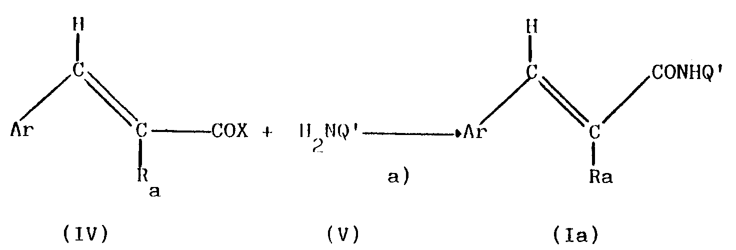
wherein the symbols Ar and Rₐ have the previously defined meanings, X represents an hydroxy group, a halogen atom and OCOR', where R' has the previously described meaning, and Q' represents a bicyclic octylamine dicyclic radical selected between the tropyl and quinuclidinyl radicals having respectively the structures: 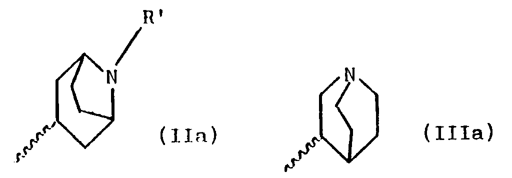
The compounds (Ia) may be optionally oxidized afterwards with an excess of a suitable oxiding agent to supply the corresponding oxides (I_{b}) 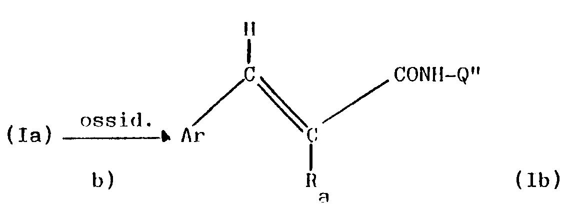
wherein the symbols Ar and Rₐ have the above defined meanings and Q'' represents a bicyclic octylamine oxidized radical, selected between the tropyl and quinuclidinyl radicals having respectively the structures 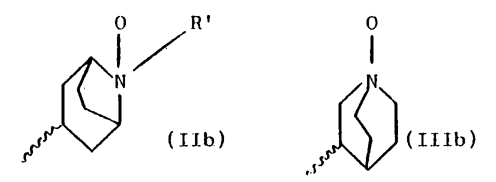
In case X represents the hydroxy group, the reaction a) is carried out in the presence of a suitable agent activating the carboxylic group, such as dicyclohexylcarbodiimide, ethoxyacetylene and other groups selected among the activating agents known in the art.

The N₂NQ' bicycloctylamine may be made to react either as free bases or as corresponding salts, in which case the presence of an organic or inorganic base such as trialkylamine, bicarbonate, carbonate, dibasic phosphate and other bases selected among the bases known in the art is required.

The H₂NQ' bicycloctylamines are known in the art: quinuclidinylamine is commercially available; tropylamine may be prepared according to the methods described in literature, see for example in EP 0013138 (20.12.79) and Helv. Chim. Acta 38, 567 (1955).

The compounds in formula IV in which X represents an halogen atom, in particular a chlorine atom, are compounds well known in the art and easily found in commerce or they can be prepared starting from the corresponding acrylic acid by reaction with a halogen agent such as thionyl chloride, phosphorus trichloride, phosphoruspentachloride, carbon tetrachloride, tryphenylphosphine and other agents of common use in the art.

The compounds of formula (IV) in which X represents the alkylcarboxylic group OCOR' are prepared from the corresponding acrylic acid by reaction with a suitable alkylchlorocarbonate in the presence of an organic or inorganic base.

The a) reaction is carried out in halogenated solvents at a temperature of -5° to 30°C, preferably first at a low temperature and then at room temperature.

The preferred solvents are halogenated hydrocarbons such as chloroform and dichloromethane, ethers such as diethylether, diisopropylether and tetrahydrofuran, esters such as ethyl formate, ethyl acetate, methyl acetate, nitrile such as acetonitrile and amides such as dimethylacetamide, dimethyl formamide and their mixtures.

The b) reaction is carried out at room temperature in the presence of an excess of a suitable oxiding agent, among which hydrogen peroxide is particularly preferred.

The compounds of the invention are particularly useful as antitussive agents; the antitussive effect is characterized by the absence of effects on the central nervous system and is totally free of addictivity phenomena.

To obtain the desired pharmacological effects, both in human and veterinary therapy, the compounds of the invention may be administered by oral route, by infusion, aerosol or parenterally, for example by intramuscular or intravenous injection. Such compounds may be administered in pure form and/or in a pharmaceutical composition.

The most suitable pharmaceutical compositions may be obtained according to the known techniques, for example the ones described in "Remington's Pharmaceutical Sciences Handbook", Hack Publishing Co, U.S.A.

A suitable dose of the compounds of the formula (I), effective as antitussive agent, varies in accordance to the nature and intensity of the tussive stimulus, to the administration route, to the age, to the weight and to the condition of the patient.

The quantity of the active principle to be administered by oral route may vary between 0.1 mg/kg/die to 300 mg/kg/die, preferably from 1 mg/kg/die to 100 mg/kg/die.

For example a suitable dose for the oral administration may contain from 0.01 to 1g of active principle.

The compounds of the invention may be administered in a single dose once a day, but spaced out and/or repeated administrations may be more convenient in accordance to the condition of the patient, to the route of administration and to the dosage used.

For the oral administration, the compounds may be formulated as solid or liquid compositions, as capsules, pills, suspensions, emulsions, syrups, drops or in granulated form soluble in water using the conventional excipients suitable for these kind of formulations. The solid unitary dose may be constituted by a solid capsule and/or of soft gelatin containing lubricants and inert excipients such as lactose, saccharose, fructose, sweetenings, aroma and starch.

For the parenteral administration, the compounds may be prepared in injectable formulations by dissolving them in physiologically acceptable diluents; a vehicle may, for example, be sterile water with or without addition of other excipients. Generally as vehicle for injectable solutions water may be used, water solutions of mineral salts, water solutions of dextrose or of other sugars, ethanol, glycols, such as for example propylene glycol and polyethylene glycol.

The compounds may also be administered as suppository, mixed with conventional vehicles such as for example cocoa butter, waxes, polyvinylpyrrolidone and/or polyethylene glycols or their derivatives.

The preferred way of administration of the compounds of the invention is the oral route.

The invention is further described but not limited by the following Examples.

### Example 1

### N-(1-Aza-bicyclo[2,2,2,]oct-3-yl)cinnamamide

Milliliters 5.5 of triethylamine are added to a suspension constituted by 2.9 g of 1-aza-bicyclo[2,2,2,]oct-3-yl amine dihydrochloride in 60 ml of chloroform; after half an hour of vigorous stirring an opalescent solution is obtained which is cooled to 0-5°C. Keeping the temperature unchanged, a solution formed of 3 g of cinmamoyl chloride in 60 ml of chloroform is added dropwise in the obtained solution. The mixture is allowed to react for 2 hours at 0-5°C and at room temperature for one night. At the end 50 ml of deionized water are added and the phases are separated. The organic phase is made anyhydrous over sodium sulphate, evaporated and the residue crystallized from ethylacetate. Grams 2.5 of N-(1-aza-bicyclo[2,2,2]oct-3-yl)cinnamamide melting at 200-202°C are obtained.

Following the procedure described above are obtained:
N-(endo-8-methyl-8-aza-bicyclo[3,2,1]oct-3-yl)cinnamamide melting over 280°C
N-(endo-8-methyl-8-aza-bicyclo[3,2,1]oct-3-yl)-4-methoxycinnamamide hydrochloride melting over 280°C.
N-(1-aza-bicyclo[2,2,2]oct-3-yl)-2-methoxycinnamamide melting at 189-191°C
N-(1-aza-bicyclo[2,2,2]oct-3-yl)-3-methoxycinnamamide melting at 145-147°C
N-(1-aza-bicyclo[2,2,2]oct-3-yl)-4-methoxycinnamamide melting at 142-145°C
N-(1-aza-bicyclo[2,2,2]oct-3-yl)-4-hydroxycinnamamide melting at 280°C
N-(1-aza-bicyclo[2,2,2]oct-3-yl)-2-chlorocinnamamide melting at 218-221°C
N-(1-aza-bicyclo[2,2,2]oct-3-yl)-3-chlorocinnamamide melting at 130-134°C
N-(1-aza-bicyclo[2,2,2]oct-3-yl)-4-chlorocinnamamide melting at 179-182°C
N-(1-aza-bicyclo[2,2,2]oct-3-yl)-2-fluorocinnamamide melting at 194-196°C
N-(1-aza-bicyclo[2,2,2]oct-3-yl)-3-fluorocinnamamide hydrochloride melting at 246-249°C
N-(1-aza-bicyclo[2,2,2]oct-3-yl)-4-fluorocinnamamide melting at 207-209°C
N-(1-aza-bicyclo[2,2,2]oct-3-yl)-α-methylcinnamamide hydrochloride melting at 244-246°C
N-(1-aza-bicyclo[2,2,2]oct-3-yl)-4-acetoxycinnamamide hydrochloride melting at 162-168°C

### Example 2

### N-(1-aza-bicyclo[2,2,2]oct-3-yl)-cinnamamide

Grams 0.30 of carbonyldiimidazole are added to a solution formed by 0.25 g of cinnamic acid in 3 ml of tetrahydrofuran.

Effervescence is formed which lasts for about 15 minutes. The so obtained cinnamoylimidazolide solution is added to a solution of 0.21 g of N-(1-aza-bicyclo[2,2,2]oct-3-yl)amine in 3 ml of tetrahydrofuran cooled to 0-5°C. It is allowed to react for 3 hours at 0°C and overnight at room temperature. The solvent is then evaporated and the residue obtained dissolved in 15 ml 2N hydrochloric acid and washed 3 times with 10 ml of chloroform. The aqueous acid phase is then adjusted to pH 9 with 2N sodium hydroxyde and extracted 3 times with 10 ml of chloroform. The organic phases are made anhydrous on sodium sulphate, concentrated and the so obtained residue is crystallized from ethylacetate. Grams 0.19 of N-(1-aza-bicyclo[2,2,2]oct-3-yl)cinnamamide melting at 200-202°C are obtained.

### Example 3

### N-(1-aza-bicyclo[2,2,2]oct-3-yl)-2-fluorocinnamamide

Grams 0.25 of 2-fluorocinnamic acid in 2 ml of tetrahydrofuran is added to a solution consisting of 0.34 g of dicyclohexylcarbodiimide and 0.19 g of N-(1-aza-bicyclo[2,2,2]oct-3-yl)amine in 2 ml of tetrahydrofuran. The temperature of the reaction is brought to 60°C and the mixture is left to react for one hour. The dicyclohexylurea which formed is then filtered, the solvent concentrated and the obtained residue dissolved in 15 ml of 2N hydrochloric acid and washed 3 times with 10 ml of chloroform. The aqueous acid phase is then adjusted to pH 9 with 2N sodium hydroxyde and again extracted 3 times with 10 ml of chloroform. The organic phases are made anhydrous on sodium sulphate, concentrated and the so obtained residue crystallized from ethylacetate. Grams 0.20 of N-(1-aza-bicyclo[2,2,2]oct-3-yl)-2-fluorocinnamamide melting at 194-196°C are obtained.

### Example 4

### N-(1-aza-bicyclo[2,2,2]oct-3-yl)-4-methoxycinnamamide.

Grams 0.17 of ethylchloroformate dissolved in 2 ml of tetrahydrofuran are added to a solution, cooled at 0°C, of 0.25 g of 4-methoxycinnamic acid and of 0.20 g of tetraethylamine in 2 ml of tetrahydrofuran. After 30 minutes the triethylamine hydrochloride formed is removed by filtration and the so obtained solution, which contains the 4-methoxycinnamic acid mixed anhydride, is added to a solution, cooled at 0°C, of 0.18 g of N-(1-aza-bicyclo[2,2,2]oct-3-yl)amine in 5 ml of chloroform. At the end of the addition the reaction mixture is kept for two hours at room temperature, then the solvent is evaporated and the so obtained residue is dissolved in 15 ml of hydrochloric acid and washed three times with 10 ml of chloroform. The aqueous acid phase is then adjusted to pH 9 with sodium hydroxyde and extracted three times with 10 ml of chloroform.

The organic phases are made anhydrous over sodium sulphate, concentrated and the obtained residue is crystallized from ethylacetate. Grams 0.22 of N-(1-aza-bicyclo[2,2,2]oct-3-yl)-4-methoxycinnamamide melting at 142-145°C are obtained.

### Example 5

### N-(1-aza-bicyclo[2,2,2]oct-3-yl)-4-chlorocinnamamide

Grams 0.31 of N-hydroxysuccinimide are added to a solution of 0.5 g of 4-chlorocinnamic acid and of 0.62 g of dicyclohexylcarbodiimide in 6 ml of tetrahydrofuran. The reaction is kept at room temperature overnight. The formed dicyclohexylurea is filtered and the solvent evaporated. The so obtained residue is dissolved in 15 ml of chloroform and washed with a saturated potassium carbonate solution. The organic phase is made anhydrous over sodium sulphate, concentrated and the residue obtained, which contains 0.75 g of 4-chlorocinnamic acid succinimido ester is dissolved in 2 ml of chloroform and added to a solution, cooled at 0°C, of 0.54 g of N-(1-aza-bicyclo[2,2,2]oct-3-yl)amina dihydrochloride and 0.55 g of tetraethylamine in 8 ml of chloroform. At the end of the addition the reaction mixture is brought to room tmperature and it is left to react over night. The organic phase is then extracted with 15 ml of 2N hydrochloric acid and the aqueous acid phase is first washed three times with 10 ml of chloroform and then adjusted to pH 9 with 2N sodium hydroxide. The basic aqueous phase is then extracted three times with 10 ml of chloroform, the organic phases are made anhydrous over sodium sulphate, concentrated and the obtained residue is crystallized from ethylacetate. Grams 0.31 of N-(1-aza-bicyclo[2,2,2]oct-3-yl)-4- chlorocinnamamide melting at 180-182°C are obtained.

### Example 6

### N-(1-aza-bicyclo[2,2,2]oct-3-yl)-4-hydroxycinnamamide

A portion of 1.8 g of N-(1-aza-bicyclo[2,2,2]oct-3-yl)-4-acetoxycinnamamide, obtained according to Example 1, is hydrolyzed at room temperature with a 10% potassium carbonate aqueous solution. After one hour the reaction mixture is adjusted to pH 8 with 2N hydrochloric acid, and the crystallized product gives 1.3 g of N-(1-aza-bicyclo[2,2,2]oct-3-yl)-4-hydroxycinnamamide melting over 280°C.

### Example 7

### N-(1-aza-bicyclo[2,2,2]oct-3-yl)-4-aminocinnamamide

A warm solution in ethanol of N-(1-aza-bicyclo[2,2,2]oct-3-yl)-4-nitrocinnamamide, obtained according to one of the previously described procedures, is added to a warm solution of ferrous sulphate heptahydrate in deionized water and concentrated ammonia. Afterwards N-(1-aza-bicyclo[2,2,2]oct-3-yl)-4-aminocinnamamide is obtained.

### Example 8

### N-(1-aza-bicyclo[2,2,2]oct-3-yl)-4-acetamidocinnamamide

A solution of N-(1-aza-bicyclo[2,2,2]oct-3-yl)-4-aminocinnamamide, obtained according to Example 7, is reacted at 0°C with acetylchloride in the presence of triethylamine to give N-(1-aza-bicyclo[2,2,2]oct-3-yl)-4-acetamidocinnam- amide.

Following the procedure described in the previous Examples the following compounds are obtained:
N-(1-aza-bicyclo[2,2,2]oct-3-yl)-4-phenoxycinnamamide
N-(1-aza-bicyclo[2,2,2]oct-3-yl)-3-phenoxycinnamamide
N-(1-aza-bicyclo[2,2,2]oct-3-yl)-trans-3-(3-pyridyl) acrylamide
N-(1-aza-bicyclo[2,2,2]oct-3-yl)-trans-3-(2-pyridyl) acrylamide
N-(1-aza-bicyclo[2,2,2]oct-3-yl)-trans-3-(4-pyridyl) acrylamide
N-(1-aza-bicyclo[2,2,2]oct-3-yl)-3-bromocinnamamide melting at 158-161°C
N-(1-aza-bicyclo[2,2,2]oct-3-yl)-4-bromocinnamamide melting at 174-176°C
N-(1-aza-bicyclo[2,2,2]oct-3-yl)-α-phenylcinnamamide hydrochloride melting at 280-282°C (with decomposition)
N-(1-aza-bicyclo[2,2,2]oct-3-yl)-3,4-dichlorocinnamamide melting at 168-170°C
N-(1-aza-bicyclo[2,2,2]oct-3-yl)-3,4-dimethoxycinnamamide
N-(1-aza-bicyclo[2,2,2]oct-3-yl)-3,4,5-trimethoxycinnamamide
N-(1-aza-bicyclo[2,2,2]oct-3-yl)-3-trifluoromethyl- cinnamamide melting at 194-196°C
N-(1-aza-bicyclo[2,2,2]oct-3-yl)-4-hydroxy-α-cyanocinnamamide
N-(1-aza-bicyclo[2,2,2]oct-3-yl)-3-hydroxy-α-cyanocinnamamide
N-(1-aza-bicyclo[2,2,2]oct-3-yl)-4-acetoxy-α-cyanocinnamamide
N-(1-aza-bicyclo[2,2,2]oct-3-yl)-3-acetoxy-α-cyanocinnamamide
N-(1-aza-bicyclo[2,2,2]oct-3-yl)-4-methoxy-α-cyanocinnamamide
N-(1-aza-bicyclo[2,2,2]oct-3-yl)-3-methoxy-α-cyanocinnamamide
N-(1-aza-bicyclo[2,2,2]oct-3-yl)-α-cyanocinnamamide
N-(endo-8-methyl-8-aza-bicyclo[3,2,1]oct-3-yl)-2-methoxycinnamamide
N-(endo-8-methyl-8-aza-bicyclo[3,2,1]oct-3-yl)-3-methoxycinnamamide
N-(endo-8-methyl-8-aza-bicyclo[3,2,1]oct-3-yl)-4-methoxycinnamamide
N-(endo-8-methyl-8-aza-bicyclo[3,2,1]oct-3-yl)-4-hydroxycinnamamide
N-(endo-8-methyl-8-aza-bicyclo[3,2,1]oct-3-yl)-2-chlorocinnamamide
N-(endo-8-methyl-8-aza-bicyclo[3,2,1]oct-3-yl)-3-chlorocinnamamide
N-(endo-8-methyl-8-aza-bicyclo[3,2,1]oct-3-yl)-4-chlorocinnamamide
N-(endo-8-methyl-8-aza-bicyclo[3,2,1]oct-3-yl)-2-fluorocinnamamide
N-(endo-8-methyl-8-aza-bicyclo[3,2,1]oct-3-yl)-3-fluorocinnamamide
N-(endo-8-methyl-8-aza-bicyclo[3,2,1]oct-3-yl)-4-fluorocinnamamide
N-(endo-8-methyl-8-aza-bicyclo[3,2,1]oct-3-yl)-α-methylcinnamamide
N-(endo-8-methyl-8-aza-bicyclo[3,2,1]oct-3-yl)-4-phenoxycinnamamide
N-(endo-8-methyl-8-aza-bicyclo[3,2,1]oct-3-yl)-3-phenoxycinnamamide
N-(endo-8-methyl-8-aza-bicyclo[3,2,1]oct-3-yl)-trans-3-(3-pyridyl)acrylamide
N-(endo-8-methyl-8-aza-bicyclo[3,2,1]oct-3-yl)-trans-3-(2-pyridyl)acrylamide
N-(endo-8-methyl-8-aza-bicyclo[3,2,1]oct-3-yl)-trans-3-(4-pyridyl)acrylamide
N-(endo-8-methyl-8-aza-bicyclo[3,2,1]oct-3-yl)-3-bromocinnamamide
N-(endo-8-methyl-8-aza-bicyclo[3,2,1]oct-3-yl)-4-bromocinnamamide
N-(endo-8-methyl-8-aza-bicyclo[3,2,1]oct-3-yl)-α-phenylcinnamamide
N-(endo-8-methyl-8-aza-bicyclo[3,2,1]oct-3-yl)-3,4-dichlorocinnamamide
N-(endo-8-methyl-8-aza-bicyclo[3,2,1]oct-3-yl)-3,4-dimethoxycinnamamide
N-(endo-8-methyl-8-aza-bicyclo[3,2,1]oct-3-yl)-3,4,5-trimethoxycinnamamide
N-(endo-8-methyl-8-aza-bicyclo[3,2,1]oct-3-yl)-4-hydroxy-α-cyanocinnamamide
N-(endo-8-methyl-8-aza-bicyclo[3,2,1]oct-3-yl)-3-hydroxy-α-cyanocinnamamide
N-(endo-8-methyl-8-aza-bicyclo[3,2,1]oct-3-yl)-4-acetoxy-α-cyanocinnamamide
N-(endo-8-methyl-8-aza-bicyclo[3,2,1]oct-3-yl)-3-acetoxy-α-cyanocinnamamide
N-(endo-8-methyl-8-aza-bicyclo[3,2,1]oct-3-yl)-4-methoxy-α-cyanocinnamamide
N-(endo-8-methyl-8-aza-bicyclo[3,2,1]oct-3-yl)-3-methoxy-α-cyanocinnamamide

### Example 9

### N-(1-aza-1-oxodobicyclo[2,2,2]oct-3-yl)-3-chloro- cinnamamide monohydrate

To a solution of 0.5 g of N-(1-aza-bicyclo[2,2,2]oct-3-yl)-3-chlorocinnamamide, obtained in accordance to Example 1, in 2 ml of ethanol, are added 0.4 ml of a solution of 35% hydrogen peroxide and stirring is maintained for 5 hours at room temperature. The excess of hydrogen peroxide is decomposed with palladium on charcoal, then the reaction mixture is filtered on celite, evaporated and treated with ethylacetate to give 0.4 g of N-(1-aza-1-oxo-bicyclo[2,2,2]oct-3-yl)-3- chlorocinnamamide monohydrate, melting at 268-271°C.

Operating in the same manner as to the above described procedure are obtained.
N-(endo-8-methyl-8-aza-8-oxobicyclo[3,2,1]oct-3-yl)-cinnamamide
N-(endo-8-methyl-8-aza-8-oxobicyclo[3,2,1]oct-3-yl)-4-methoxycinnamamide
N-(1-aza-1-oxobicyclo[2,2,2]oct-3-yl)-2-methoxycinnamamide
N-(1-aza-1-oxobicyclo[2,2,2]oct-3-yl)-3-methoxycinnamamide
N-(1-aza-1-oxobicyclo[2,2,2]oct-3-yl)-4-methoxycinnamamide
N-(1-aza-1-oxobicyclo[2,2,2]oct-3-yl)-4-hydroxycinnamamide
N-(1-aza-1-oxydobicyclo[2,2,2]oct-3-yl)chlorocinnamamide
N-(1-aza-1-oxobicyclo[2,2,2]oct-3-yl)-3-chlorocinnamamide
N-(1-aza-1-oxobicyclo[2,2,2]oct-3-yl)-4-chlorocinnamamide
N-(1-aza-1-oxobicyclo[2,2,2]oct-3-yl)-2-fluorocinnamamide
N-(1-aza-1-oxobicyclo[2,2,2]oct-3-yl)-3-fluorocinnamamide
N-(1-aza-1-oxobicyclo[2,2,2]oct-3-yl)-4-fluorocinnamamide
N-(1-aza-1-oxobicyclo[2,2,2]oct-3-yl)-α-methylcinnamamide
N-(1-aza-1-oxobicyclo[2,2,2]oct-3-yl)-4-acetoxycinnamamide
N-(1-aza-1-oxobicyclo[2,2,2]oct-3-yl)-4-phenoxycinnamamide
N-(1-aza-1-oxobicyclo[2,2,2]oct-3-yl)-3-phenoxycinnamamide
N-(1-aza-1-oxobicyclo[2,2,2]oct-3-yl)-trans-3-(3-pyridyl)-acrylamide
N-(1-aza-1-oxobicyclo[2,2,2]oct-3-yl)-trans-3-(2-pyridyl)-acrylamide
N-(1-aza-1-oxobicyclo[2,2,2]oct-3-yl)-trans-3-(4-pyridyl)-acrylamide
N-(1-aza-1-oxobicyclo[2,2,2]oct-3-yl)-3-bromocinnamamide
N-(1-aza-1-oxobicyclo[2,2,2]oct-3-yl)-4-bromocinnamamide
N-(1-aza-1-oxobicyclo[2,2,2]oct-3-yl)-α-phenylcinnamamide
N-(1-aza-1-oxobicyclo[2,2,2]oct-3-yl)-3,4-dichloro- cinnamamide
N-(1-aza-1-oxobicyclo[2,2,2]oct-3-yl)-3,4-dimethoxycinnamamide
N-(1-aza-1-oxobicyclo[2,2,2]oct-3-yl)-3,4,5-trimethoxycinnamamide
N-(endo-8-methyl-8-aza-8-oxobicyclo[3,2,1]oct-3-yl)-2-methoxycinnamamide
N-(endo-8-methyl-8-aza-8-oxobicyclo[3,2,1]oct-3-yl)-3-methoxycinnamamide
N-(endo-8-methyl-8-aza-8-oxobicyclo[3,2,1]oct-3-yl)-4-methoxycinnamamide
N-(endo-8-methyl-8-aza-8-oxobicyclo[3,2,1]oct-3-yl)-4-hydroxycinnamamide
N-(endo-8-methyl-8-aza-8-oxobicyclo[3,2,1]oct-3-yl)-2-chlorocinnamamide
N-(endo-8-methyl-8-aza-8-oxobicyclo[3,2,1]oct-3-yl)-3-chlorocinnamamide
N-(endo-8-methyl-8-aza-8-oxobicyclo[3,2,1]oct-3-yl)-4-chlorocinnamamide
N-(endo-8-methyl-8-aza-8-oxobicyclo[3,2,1]oct-3-yl)-2-fluorocinnamamide
N-(endo-8-methyl-8-aza-8-oxobicyclo[3,2,1]oct-3-yl)-3-fluorocinnamamide
N-(endo-8-methyl-8-aza-8-oxobicyclo[3,2,1]oct-3-yl)-4-fluorocinnamamide
N-(endo-8-methyl-8-aza-8-oxobicyclo[3,2,1]oct-3-yl)-α-methylcinnamamide
N-(endo-8-methyl-8-aza-8-oxobicyclo[3,2,1]oct-3-yl)-4-phenoxycinnamamide
N-(endo-8-methyl-8-aza-8-oxobicyclo[3,2,1]oct-3-yl)-3-phenoxycinnamamide
N-(endo-8-methyl-8-aza-8-oxobicyclo[3,2,1]oct-3-yl)-3-bromocinnamamide
N-(endo-8-methyl-8-aza-8-oxobicyclo[3,2,1]oct-3-yl)-4-bromocinnamamide
N-(endo-8-methyl-8-aza-8-oxobicyclo[3,2,1]oct-3-yl)-α-phenylcinnamamide
N-(endo-8-methyl-8-aza-8-oxobicyclo[3,2,1]oct-3-yl)-3,4-dichlorocinnamamide
N-(endo-8-methyl-8-aza-8-oxobicyclo[3,2,1]oct-3-yl)-3,4-dimethoxycinnamamide
N-(endo-8-methyl-8-aza-8-oxobicyclo[3,2,1]oct-3-yl)-3,4,5-trimethoxycinnamamide

## Claims

1. Amide derivatives having the structure formula 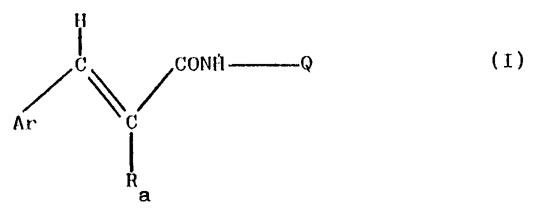 wherein
Ar represents a pyridyl radical or a benzene ring which optionally may be substituted with one or more halogen atoms or one or more groups selected among lower alkyl, lower haloalkyl, lower alkoxy, phenoxy, hydroxy, acyloxy, amino, acylamino and nitro,
Rₐ represents hydrogen, lower alkyl, phenyl or cyano;
Q represents a bicyclic octylamino radical selected among the radicals having respectively the structures 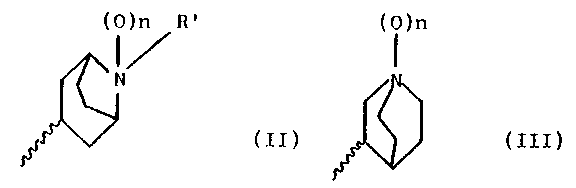
wherein
R' represents a lower alkyl radical and
n represents 0 or 1, provided that n=0 when Rₐ is cyano and/or Az is pyridyl
in their exo or endo configuration or mixture thereof and the corresponding pharmaceutically acceptable non toxic addition salts.

2. Amide derivatives according to claim 1, characterized by the fact that Ar represents a benzene radical which may be optionally substituted with one or more halogen atoms or one or more groups selected among lower alkyl, lower haloalkyl, lower alkoxy, phenoxy, hydroxy, acyloxy, amino, acylamino and nitro.

3. Amide derivatives according to claim 1 characterized by the fact that Ar represents a pyridyl radical which may be optionally substituted with one or more halogen atoms or one or more groups selected among lower alkyl, lower haloalkyl, lower alkoxy, phenoxy, hydroxy, acyloxy, amino, acylamino and nitro.

4. Amide derivatives according to claim 2, characterized by the fact that Q represents the β-tropyl radical.

5. Amide derivatives according to claim 2, characterized by the fact that Q is selected among R, S and RS quinuclidinyl radical.

6. N-(1-Aza-bicyclo[2,2,2]oct-3-yl)-2-methoxycinnamamide.

7. N-(1-Aza-bicyclo[2,2,2]oct-3-yl)-4-methoxycinnamamide.

8. N-(1-Aza-bicyclo[2,2,2]oct-3-yl)-α-methylcinnamamide.

9. N-(1-Aza-bicyclo[2,2,2]oct-3-yl)-2-fluorocinnamamide.

10. Pharmaceutical composition having antitussive activity which contain a therapeutically active amount of one or more compounds according to claims 1-9 in admixture with suitable farmaceutically acceptable diluents.
